# EUROPÄISCHE PATENTSCHRIFT

(11) **EP 0 318 762 B1**
(45) Veröffentlichungstag und Bekanntmachung des Hinweises auf die Patenterteilung: **25.08.1993**
(21) Anmeldenummer: 88119107.6
(22) Anmeldetag: 17.11.1988
(51) Int. Cl.: A61B 17/58

(54) **Osteosynthetische Druckplatte**
Osteosynthetic pressure plate
Plaque de compression ostéosynthétique

(30) Priorität: 02.12.1987 CH 4710/87
(43) Veröffentlichungstag der Anmeldung: 07.06.1989
(73) Patentinhaber: Synthes AG, Chur, CH-7002 Chur (CH)
(72) Erfinder: Klaue, Kaj, Dr., CH-3028 Spiegel (CH); Brunner, Hans, Dr., CH-4437 Waldenburg (CH); Perren, Stephan M., Prof., CH-7260 Davos Dorf (CH)
(74) Vertreter: Lusuardi, Werther Giovanni, Dr.

(56) Entgegenhaltungen:
- EP-A- 0 217 085
- CH-A- 462 375
- CH-A- 651 192
- DE-A- 3 442 004

## Beschreibung

Die Erfindung bezieht sich auf eine osteosynthetische Druckplatte mit mehreren in der Längsachse der Druckplatte angeordneten Löchern zur Aufnahme von Knochenschrauben.

Osteosynthetische Druckplatten dieser Art sind seit längerem bekannt, beispielsweise aus der CH-A5 462′375. Aufgrund neuer Erkenntnisse wurden vielfältige Verbesserungen dieser Druckplatte vorgeschlagen. Dabei beschränkte man sich jedoch immer nur auf die Modifikation einzelner konstruktiver Merkmale, welche zwar in vielen Fällen graduelle Verbesserungen gegenüber dem bekannten Stand der Technik brachten, jedoch kaum einen wirklichen innovativen Sprung bedeuteten. Dies ist wohl der Grund weshalb auch heute noch der weitaus grösste Teil der klinisch verwendeten Druckplatten herkömmlicher Art sind.

Hier will die Erfindung Abhilfe schaffen. Der Erfindung liegt die Aufgabe zugrunde durch eine optimale Auswahl aufeinander abgestimmter konstruktiver Merkmale die Applikation der Druckplatte und deren Integrität intraoperativ zu verbessern, das Knochenwachstum unter der Druckplatte postoperativ zu fördern und eine dynamische Kompressionswirkung zu gewährleisten.

Die Erfindung löst die gestellte Aufgabe mit einer osteosynthetischen Druckplatte, welche die Merkmale des Anspruchs 1 aufweist.

Die durch die Erfindung erreichten Vorteile sind im wesentlichen darin zu sehen, dass dank der Erfindung einerseits die Anpassung der Druckplatte an die Anatomie des Knochens leichter fällt und anderseits die Vaskularisierung und damit das Knochenwachstum unter der Druckplatte erheblich verbessert ist. Ueberraschenderweise wurde gefunden, dass nach Entfernung von implantierten erfindungsgemässen Druckplatten das Plattenbett, d.h. die knöcherne Platteneinbettung als Reaktion des Knochens auf das Vorhandensein der implantierten Platte, nicht wie bei herkömmlichen Platten scharfkantig, sondern abgerundet ausgebildet war. Dadurch kann die Entfernung der erfindungsgemässe Platte nach abgeschlossener Knochenbruchheilung einfacher, d.h. ohne Zuhilfenahme von Instrumenten und ohne Zerstörung der neugebildeten Knochenlamellen des Plattenbettes erfolgen. Die unbeschädigt gebliebenen Knochenlamellen verstärken im Frakturgebiet den Knochenquerschnitt und reduzieren dadurch die Gefahr einer Refraktur.

Eine weitere Verbesserung kann dadurch erreicht werden, dass das vorzugsweise als Kreisringsektor ausgebildete Querschnittsprofil im Bereich zwischen den Bohrlöchern flächenmässig derart reduziert ist, dass der Biegewiderstand in diesem Bereich gleich gross, vorzugsweise jedoch kleiner ist als im Bereich der Löcher. Wegen des dadurch erreichten gleichmässigen, im Zwischenlochbereich vorzugsweise geringeren, Biegewiderstandes kann die Platte leichter an die Anatomie des Knochens angepasst werden. Im weiteren kann die Anpassung der Platte ohne Deformation der Löcher erfolgen, so dass der resultierende Verlust an Ermüdungsfestigkeit und an Passgenauigkeit der Schraubenköpfe minimal ist.
Für verschiedene Anwendungszwecke hat sich die zusätzliche Ausgestaltung der erfindungsgemässen Druckplatte als dynamische Kompressionsplatte (gemäss der CH-A5 651 192) mit mindestens einem entsprechend ausgestalteten Loch als vorteilhaft erwiesen.

Ein Ausführungsbeispiel der Erfindung, welches zugleich das Funktionsprinzip erläutert, ist in der Zeichnung dargestellt und wird im folgenden näher beschrieben. Es zeigt
Fig. 1 eine perspektivische Ansicht der erfindungsgemässen osteosynthetischen Druckplatte,
Fig. 2 eine Seitenansicht der erfindungsgemässen osteosynthetischen Druckplatte,
Fig. 3 eine Aufsicht der erfindungsgemässen osteosynthetischen Druckplatte,
Fig. 4 einen zur Längsachse der Druckplatte orthogonalen Schnitt längs der Linie IV-IV in Fig. 3,
Fig. 5 einen zur Längsachse der Druckplatte parallelen Schnitt längs der Linie V-V in Fig. 3,
Fig. 6 einen zur Längsachse der Druckplatte orthogonalen Schnitt längs der Linie VI-VI in Fig. 3,
Fig. 7 einen zu Fig. 6 analogen, orthogonalen Schnitt im Zwischenlochbereich einer modifizierten Druckplatte, und
Fig. 8 einen weiteren zu Fig. 6 analogen, orthogonalen Schnitt im Zwischenlochbereich einer modifizierten Druckplatte.

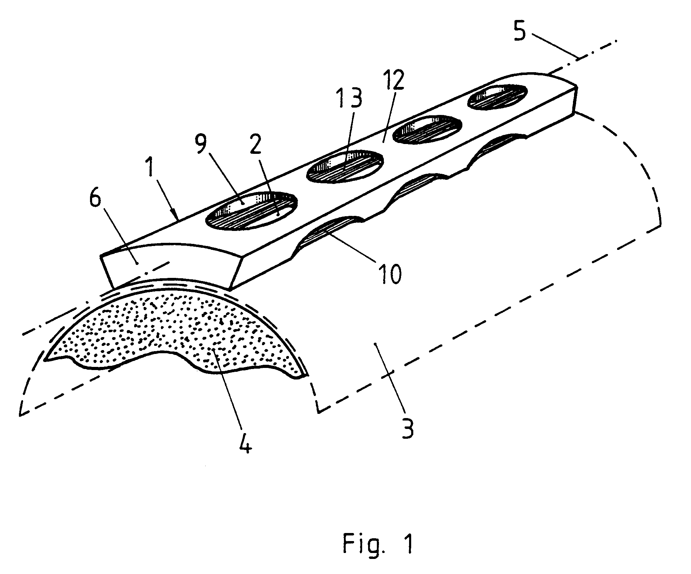

Gemäss den Fig. 1 bis 3 besteht die erfindungsgemässe osteosynthetische Druckplatte aus einer länglichen, hohlzylindersektorförmigen Druckplatte 1 mit mehreren entlang der Längsachse 5 angeordneten Löchern 2 zur Aufnahme von Knochenschrauben. Das orthogonal zur Längsachse 5 liegende Querschnittsprofil 6 der Druckplatte 1 ist derart gestaltet, dass die zur Knochenapplikationsfläche 3 parallelen Schnittflächen durch die Druckplatte 1 sich mit zunehmendem Abstand von der Knochenapplikationsfläche 3 mindestens in einem Teilbereich erweitern. Im weiteren weist die zur Auflage auf den Knochen 4 bestimmte Unterseite der Druckplatte 1 - vorzugsweise spärische - Vertiefungen 10 auf, so dass unmittelbar nach der Implantation Hohlräume zwischen Knochen und Druckplatte 1 resultieren.

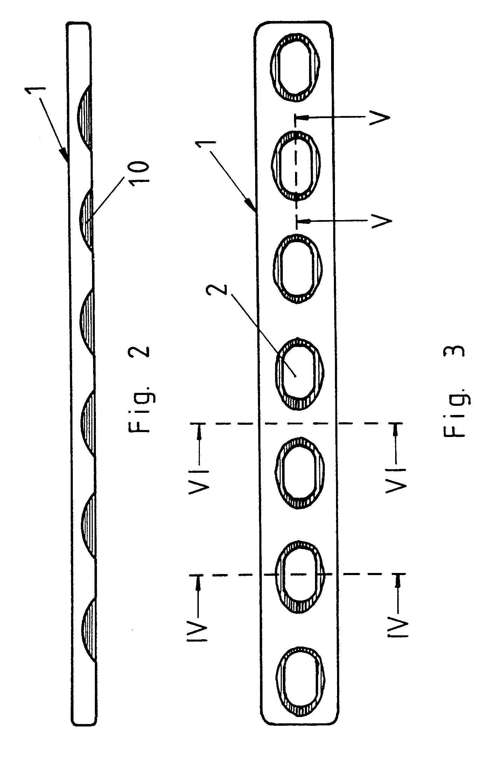

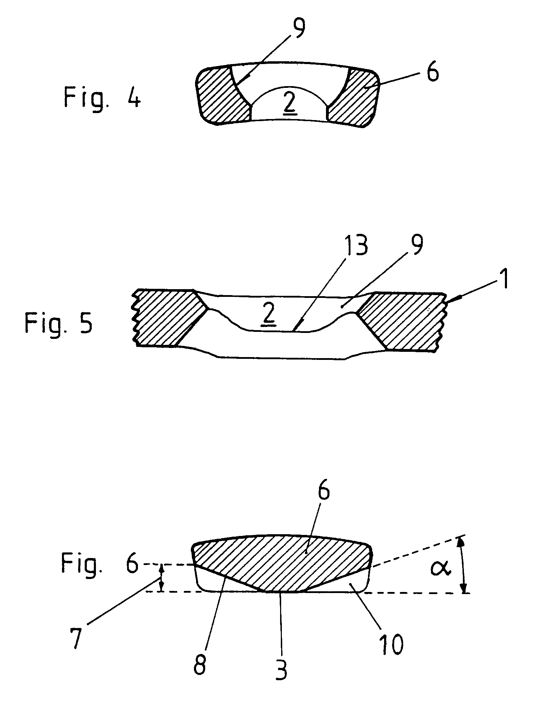

Der in Fig. 4 gezeigte Profilquerschnitt 6 auf der Höhe IV-IV der Fig. 3 ist durch das Zentrum eines Loches 2 gelegt und weist in seinem oberen Teil eine sphärische Auflagefläche 9 auf, für den darin aufzunehmenden Kugelkopf einer Knochenschraube.

Fig. 5 zeigt einen Teil-Schnitt durch die Längsachse 5 im Bereich V-V der in Fig. 3 gezeigten Druckplatte 1. Das Loch 2 weist im oberen Teil die sphärische sich verjüngende Auflagefläche 9 auf, welche sich gegen die Knochenkontaktfläche hin wieder erweitert um den Freiraum zwischen Druckplatte 1 und Knochen 4 zu vergrössern. Das symmetrisch zur Längsachse 5 angebrachte länglich ausgebildete Loch 2 besitzt an seinen längsseitigen Wandungen ein gegenüber der Oberseite 12 der Druckplatte 1 abgesenktes Widerlager 13, auf dem sich der Schraubenkopf parallel zur Längsachse 5 verschieben kann.

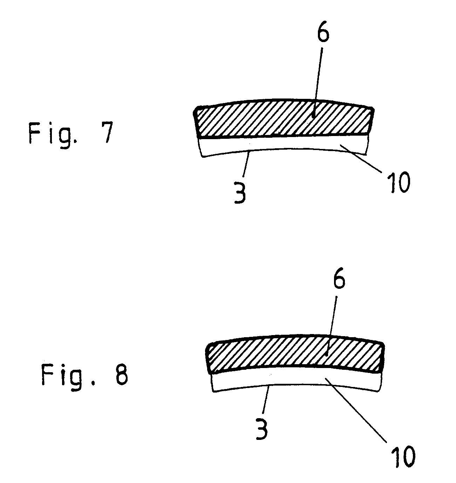

Der in Fig. 6 gezeigte Profilquerschnitt 6 verläuft auf der Höhe VI-VI der Fig. 3 im Bereich der Vertiefung 10 und zeigt die im Teilbereich 7 annähernd trapezoide Form des Profilquerschnitts zwischen zwei Löchern 2. Die Seitenflächen 8 der Druckplatte 1 im Bereich des Profilquerschnitts 6 schliessen mit der Knochenapplikationsfläche 3 einen Winkel von 20° ein . Je nach Anwendungsgebiet kann dieser Winkel innerhalb eines Bereiches von 12° - 28°, vorzugsweise von 18°- 22° variieren.

Es sind eine ganze Reihe weiterer Profilquerschnitte 6 möglich, welche alle dem gemeinsamen Prinzip einer gegenüber herkömmlichen Druckplatten reduzierten Knochenapplikationsfläche 3 und reduzierten Biegefestigkeit im Zwischenlochbereich gehorchen. Beispielsweise können sich die Vertiefungen 10 auch über die gesamte Breite der Plattenunterseite erstrecken, wie dies in den Fig. 7 und 8 dargestellt ist, wobei die Plattenunterseite in diesem reduzierten Profilbereich entweder plan, wie in Fig. 7, oder konkav wie in Fig. 8 ausgestaltet sein kann.

Die Unterseite der Druckplatte 1 kann statt konkav auch konvex gestaltet sein, z.B. mit einer ellipsoiden, paraboloiden oder ähnlichen Form, welche praktisch nur noch einen Linienkontakt mit dem Knochen zulässt.

## Patentansprüche

1. Osteosynthetische Druckplatte (1) mit mehreren in der Längsachse (5) der Druckplatte (1) angeordneten Löchern (2) zur Aufnahme von Knochenschrauben und einem orthogonal zur Längsachse (5) liegenden Querschnittsprofil (6), welches mindestens im Bereich zwischen den Löchern (2) derart gestaltet ist, dass die zur Knochenapplikationsfläche (3) parallelen Schnittflächen durch die Druckplatte (1) sich mit zunehmendem Abstand von der Knochenapplikationsfläche (3) mindestens in einem Teilbereich (7) erweitern, dadurch gekennzeichnet, dass die zur Auflage auf den Knochen (4) bestimmte Unterseite der Druckplatte (1), zusätzlich zu den Löchern (2), Vertiefungen (10) nur im Bereich zwischen den Löchern (2) aufweist.

2. Osteosynthetische Druckplatte nach Anspruch 1, dadurch gekennzeichnet, dass das vorzugsweise als Kreisringsektor ausgebildete Querschnittsprofil (6) im Bereich zwischen den Löchern (2) flächenmässig derart reduziert ist, dass der Biegewiderstand in diesem Bereich nicht grösser ist als im Bereich der Löcher (2).

3. Osteosynthetische Druckplatte nach Anspruch 1 oder 2, dadurch gekennzeichnet, dass die Druckplatte (1) mindestens ein in Richtung der Längsachse (5) länglich ausgebildetes Loch (2) aufweist, dessen längsseitige Wandungen ein gegenüber der Oberseite (12) der Druckplatte (1) abgesenktes Widerlager (13) für den Schraubenkopf bildet, auf dem sich der Schraubenkopf parallel zur Längsachse (5) verschieben kann.

4. Osteosynthetische Druckplatte nach einem der Ansprüche 1 bis 3, dadurch gekennzeichnet, dass die an die Knochenapplikationsfläche (3) anschliessenden Seitenflächen (8) der Druckplatte (1) mindestens im Bereich zwischen den Löchern (2) mit der Knochenapplikationsfläche (3) einen Winkel von 12° - 28°, vorzugsweise von 18° - 22° einschliessen.

5. Osteosynthetische Druckplatte nach einem der Ansprüche 1 bis 4, dadurch gekennzeichnet, dass mindestens der obere, der Oberseite der Druckplatte (1) zugewandte Teil des Loches (2) als sphärische Auflagefläche (9) für den darin aufzunehmenden Knochenschraubenkopf ausgebildet ist.

6. Osteosynthetische Druckplatte nach einem der Ansprüche 1 bis 5, dadurch gekennzeichnet, dass das Querschnittsprofil (6) mindestens im Teilbereich (7) eine annähernd trapezoide Form aufweist.

7. Osteosynthetische Druckplatte nach einem der Ansprüche 1 bis 6, dadurch gekennzeichnet, dass sich die Vertiefungen (10) über die gesamte Breite der Plattenunterseite erstrecken.

## Claims

1. An osteosynthetic compression plate (1) having a plurality of screw holes (2) positioned along the longitudinal axis (5) of the compression plate (1) for receiving bone screws and having a cross-section profile (6) orthogonal to the longitudinal axis (5), which at least in the region between the screw holes (2) is designed in such a way that the cross-sections through the compression plate (1) parallel to the bone application surface (3) are expanding, at least in a subdomain, with increasing distance from the bone application surface (3), characterized in that the lower surface of the compression plate (1) intended for positioning on bone (4) is provided, additionally to the screw holes (2), with recesses (10) only in the region between the screw holes (2).

2. An osteosynthetic pressure plate (1) according to claim 1, characterized in that the cross-section profile (6), preferably being a sector of an annulus, is reduced in area in the region between the screw holes (2) to the extent that the bending resistance in this region is no greater than in the region of the screw holes (2).

3. An osteosynthetic pressure plate (1) according to claim 1 or 2, characterized in that the pressure plate (1) has at least one oblong screw hole (2) with its long sides in the same direction as the longitudinal axis (5), said screw hole (2) having side walls adapted to form an abutment (13) for a screw head depressed with regard to the upper surface (12) of the pressure plate (1), enabling a screw head to slide parallel to the longitudinal axis (5).

4. An osteosynthetic pressure plate (1) according to one of the claims 1 to 3, characterized in that the lateral surfaces (8) of the pressure plate (1) adjacent to the bone application surface (3) form an angle of 12° - 28°, preferably of 18° - 22° with the bone application surface (3) at least in the region between the screw holes (2).

5. An osteosynthetic pressure plate (1) according to one of the claims 1 to 4, characterized in that at least the upper part of the screw hole (2) adjacent to the upper surface (12) of the pressure plate (1) is constructed as a spherical bearing surface (9) for receiving a screw head therein.

6. An osteosynthetic pressure plate (1) according to one of the claims 1 to 5, characterized in that the cross-section profile (6) has at least in the subdomain (7) an approximately trapezoidal shape.

7. An osteosynthetic pressure plate (1) according to one of the claims 1 to 6, characterized in that the recesses (10) are extending over the entire width of the lower surface of the pressure plate (1).

## Revendications

1. Plaque de compression pour ostéosynthèse (1) avec plusieurs trous (2) pratiqués suivant l'axe longitudinal (5) de la plaque de compression (1) pour reprendre des vis osseuses et avec un profil (6) de section transversale situé perpendiculairement par rapport à l'axe longitudinal (5) et configuré au moins dans la partie reliant deux trous (2) de manière à ce que les surfaces découpées parallèles à la surface (3) d'application sur l'os s'élargissent au moins dans une partie (7) de la plaque de compression (1) au fur et à mesure que l'on s'écarte de la surface (3) d'application sur l'os, caractérisée en ce que la face inférieure, destinée à être posée sur l'os (4), de la plaque de compression (1) présente en plus des trous (2) et uniquement dans l'espace séparant les trous (2) des renfoncements (10).

2. Plaque de compression pour ostéosynthèse selon la revendication 1, caractérisée en ce que le profil (6) de la section transversale présentant de préférence la forme d'un secteur annulaire circulaire se réduit en superficie dans la zone séparant les trous (2) de telle sorte que la résistance à la friction dans cette zone ne soit pas supérieure à celle existant au voisinage des trous (2).

3. Plaque de compression pour ostéosynthèse selon la revendication 1 ou 2, caractérisée en ce que la plaque de compression (1) présente au moins un trou (2) dont la forme est allongée dans le sens de l'axe longitudinal (5) et dont les parois allongées forment pour la tête de la vis un appui (13) situé plus bas que la face supérieure (12) de la plaque de compression (1), appui sur lequel la tête de la vis peut glisser parallèlement à l'axe longitudinal (5).

4. Plaque de compression pour ostéosynthèse selon l'une des revendications 1 à 3, caractérisée en ce que les surfaces latérales (8) se raccordant à la surface (3) d'application sur l'os, de la plaque de compression (1) forment au moins dans la zone séparant les trous (2) un angle de 12° - 28°, et de préférence 18° - 22° avec la surface (3) d'application sur l'os.

5. Plaque de compression pour ostéosynthèse selon l'une des revendications 1 à 4, caractérisée en ce qu'au moins la partie supérieure du trou 2 tournée vers la face supérieure de la plaque de compression (1) présente la forme d'une surface de pose sphérique (9) pour la tête de la vis osseuse qui y est reprise.

6. Plaque de compression pour ostéosynthèse selon l'une des revendications 1 à 5, caractérisée en ce que le profil (6) de la section transversale présente au moins dans la partie (7) une forme trapézoïdale rapprochée.

7. Plaque de compression pour ostéosynthèse selon l'une des revendications 1 à 6, caractérisée en ce que les renfoncements (10) s'étendent sur toute la largeur de la face inférieure de la plaque.
